# EUROPEAN PATENT APPLICATION

(11) **EP 1 310 209 A2**
(43) Date of publication of application: **14.05.2003**
(21) Application number: 02102496.3
(22) Date of filing: 25.10.2002
(51) Int. Cl.: A61B 3/16

(54) **Non-Contact Tonometer Having Fluid Pump Driven By Proportional Solenoid**

(30) Priority: 09.11.2001 US 37327
(71) Applicant: Leica Microsystems Inc., Buffalo, NY 14240-0123 (US)
(72) Inventor: LUCE, David A., Clarence Center, NY 14032 (US); KELKENBERG, David G., Akron, NY 14001 (US)
(74) Representative: Reichert, Werner F., Dr.

(57) **Abstract**

A non-contact tonometer comprises a fluid pump system having a linear proportional solenoid for driving a piston of the fluid pump system to generate a fluid pulse in a predetermined and controlled manner. The energizing current to the linear proportional solenoid is dictated by data stored in a digital look-up table.

## Description

The present invention relates generally to ophthalmic instruments, and more particularly to a non -contact tonometer having an improved fluid pump system for generating a fluid pulse.

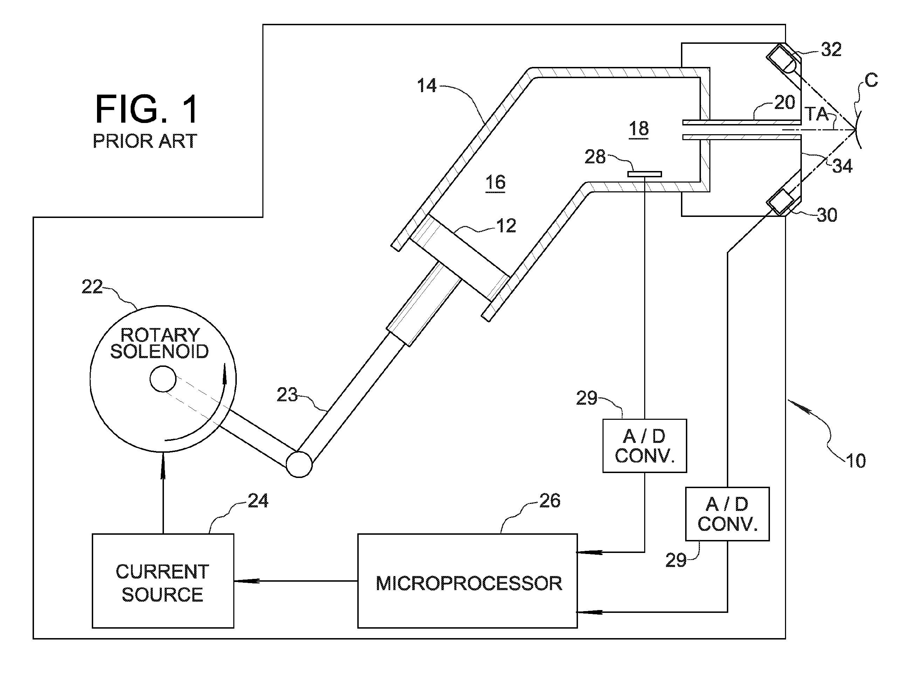

Non-contact tonometers are well -known in the field of ophthalmology for measuring intraocular pressure (IOP) by directing a fluid pulse at the cornea C to cause observable deformation of the cornea. In prior art non -contact tonometers, such as tonometer 10 shown schematically in Fig. 1, the fluid pulse is generated by a piston 12 slidably received by a cylinder housing 14 and axially driven relative to the cylinder housing to compress fluid within a compression chamber 16 defined by the cylinder housing. A plenum chamber 18 directly adjoins compression chamber 14, and a fluid discharge tube 20 is arranged in flow communication with the compression chamber by way of the plenum chamber for directing a fluid pulse along a test axis TA toward cornea C. Mea surement of IOP is based on correlation to the pressure within plenum chamber 18 at the moment a predetermined area of the cornea is flattened, a condition known as "applanation". In order to provide a signal indicative of the occurrence of applanation, a photosensitive detector 30 is positioned in a symmetrically oblique arrangement about test axis TA to receive corneally reflected light from emitter 32, whereby a peak signal is produced by detector 30 when the corneal surface is flat for coherent reflect ion.

For many years, non -contact tonometers relied exclusively on a rotary solenoid 22 connected via an arm linkage 23 to the piston 12 for driving the piston in its compression stroke. The rotary solenoid 22 was energized by a constant current source 24 under the control of a microprocessor 26, an arrangement that was preferred because it produced a linear increase in plenum pressure as a function of time. This behavior was desirable because the plenum pressure at applanation could be indirectly ascert ained in an easy fashion by observing the length of time necessary to achieve applanation. With the use of a rotary solenoid, the force acting on the piston is low at the beginning of the compression stroke and increases during the compression stroke, cau sing an unwanted delay in the fluid pump.

As pressure sensor technology progressed, the use of a miniature pressure sensor 28 in the plenum chamber 18 to directly monitor plenum pressure replaced the use of an indirect time -based pressure calculation. T he analog signals from applanation detector 30 and pressure sensor 28 are digitized by analog -to-digital converter circuits 29 and input to the microprocessor 26 for calculating IOP in a manner well-known to those skilled in the art.

Despite the fact that time-based pressure measurement has been obsolete for nearly a decade, manufacturers of non -contact tonometers have clung to the rotary solenoid as a means for driving the piston. It is also known, however, to use a linear motor for driving the piston in a non -contact tonometer. For example, use of a linear d.c. motor is taught in U.S. Patent No. 5,048,526 issued

September 17, 2001 and U.S. Patent No. 5,779,633 issued July 14, 1998. Linear motors provide reversible control of the piston movement direct ion by way of changing the direction of the energizing current in the motor coils.

U.S. Patent No. 6,159,148 to Luce teaches the use of a rotary solenoid or a linear motor in combination with an increasing, as opposed to a constant, current source. The goal of the invention described in the '148 patent is to provide a non - linear pressure increase in the plenum chamber to reduce the impulse energy associated with the pulse that is responsible for patient discomfort. While this patent teaches the desirabi lity of a non-linear pressure curve, its approach in attaining this goal is limited by difficulty in suitably controlling the output of a rotary solenoid or a standard linear d.c. motor.

Therefore, it is an object of the present invention to provide a no n-contact tonometer with a fluid pump system that is controllable with respect to the force applied to a piston thereof throughout the compression stroke of the piston.

It is another object of the present invention to provide a fluid pump system for a no n-contact tonometer that is fast to respond to energizing current.

In view of these and other objects, a non -contact tonometer of the type having a cylinder defining a compression chamber, a piston movable in a forward direction along a stroke axis relat ive to the cylinder for compressing fluid within the compression chamber, drive means operatively connected to the piston for forcing the piston in the forward direction, energizing means for supplying current to the drive means, and a fluid discharge tube in flow communication with the compression chamber for directing a fluid pulse along a test axis, is improved by providing a linear proportional solenoid in place of a rotary solenoid or standard linear motor as the drive means for the piston. The linear proportional solenoid has an output driving force that is proportional to its energizing current, thereby allowing for desired motion control throughout the compression stroke. In a preferred embodiment, the energizing current is controlled in accordance with a predefined look-up table stored in programmable memory.

The nature and mode of operation of the present invention will now be more fully described in the following detailed description of the invention taken with the accompanying drawing figures , in which:
Fig. 1 is a schematic depiction of a non -contact tonometer formed in accordance with known prior art;
Fig. 2 is a schematic depiction of a non -contact tonometer formed in accordance with the present invention;
Fig. 3 is a graph of drive curr ent versus time for the linear proportional solenoid of the present invention; and
Fig. 4 is a schematic diagram of the circuit producing the drive current shown in Fig. 3.

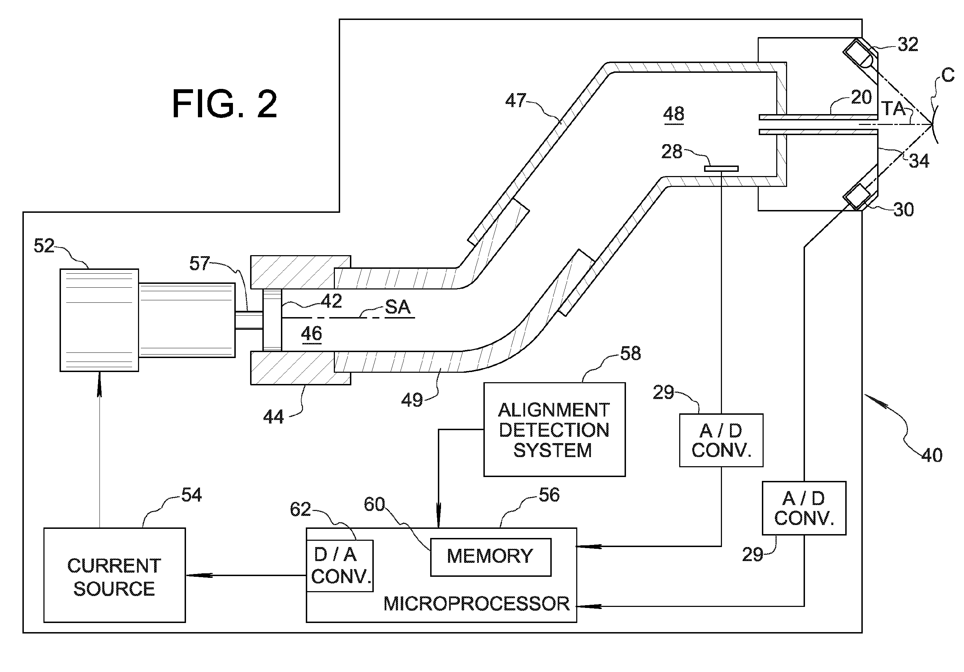

Referring to Fig. 2 of the drawings, a tonometer 40 includes a fluid pump system for generating a fluid pulse used to applanate a patient's cornea during testing. In accordance with a preferred embodiment of the present invention, the fluid pump system comprises a piston 42 axially movable relative to a cylinder 44 along a stroke ax is SA for compressing fluid within an internal compression chamber 46 defined thereby, a housing 47 defining an internal plenum chamber 48, a flow tube 49 providing a fluid conduit from compression chamber 46 to plenum chamber 48, and a fluid discharge tub e 20 mounted through the wall of housing 47 for guiding pressurized fluid from plenum chamber 48 along test axis TA directed at patient cornea C.

In accordance with the present invention, a linear proportional solenoid 52 is operatively connected to pis ton 42 for causing axially directed movement of piston 42 relative to cylinder 44. A linear proportional solenoid is a specialized type of linear motor wherein the output driving force is proportional to the energizing current, and is most often used in co nnection with control valves. Linear proportional solenoid 52 is connected to a current source 54 which supplies energizing current to the linear proportional solenoid under the control of a microprocessor 56. A suitable linear proportional solenoid is a LEDEX® Linear Shift Solenoid Part No. 197887 -001. As can be seen in Fig. 2, piston 42 is fixed for travel with a plunger 57 of linear proportional solenoid 52, as by threaded attachment or by fitted attachment with or without mechanical fasteners or adhesives.

Linear proportional solenoid 52 remains de -energized and piston 42 remains at rest until proper positioning of discharge tube 20 relative to cornea C is achieved as determined by an alignment detection system 58 connected to microprocessor 56. Ali gnment detection system 58 can be any suitable system, for example an alignment system taught in commonly owned U.S. Patent No. 4,881,807 issued November 21, 1989, the disclosure of which is hereby incorporated by reference. Once alignment is achieved, mi croprocessor 56 provides a signal used by current source 54 to provide the driving current according to a preprogrammed ramp form, as will be described below.

The use of linear proportional solenoid 52 enables programmable control of the force driving p iston 42 through the compression stroke. More specifically, a lookup table stored in a programmable memory 60 associated with microprocessor 56 includes digital information describing the desired current versus time relationship, which information can be used to actually generate the energizing current ramp.

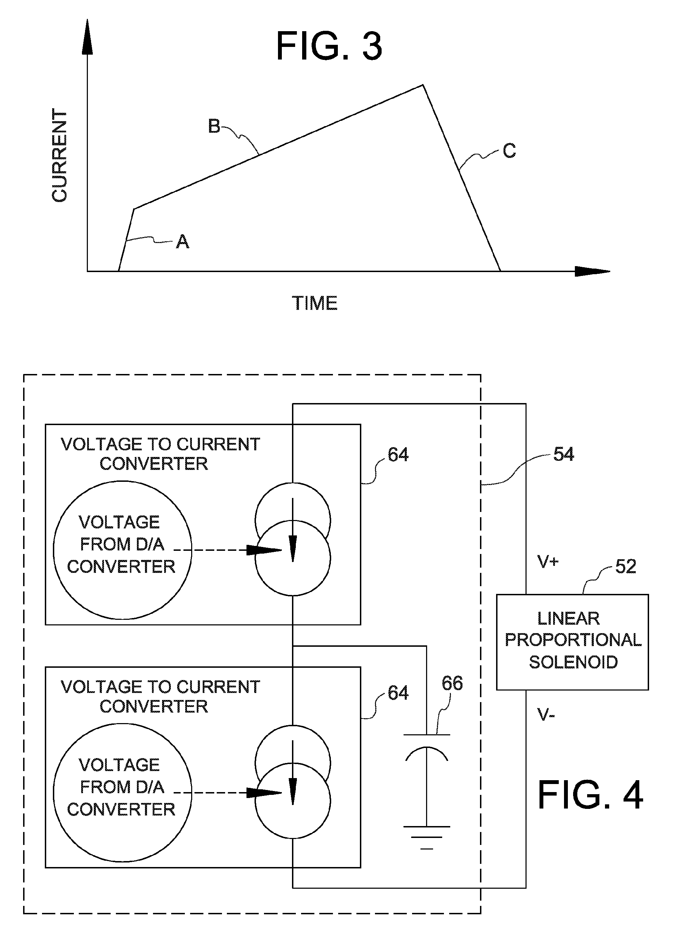

By way of non-limiting example, Fig. 3 shows a presently favored relationship of drive current versus time as generated by current source 54 for the compression stroke of piston 42. The shape includ es three stages A, B, and C each defined by a straight line segment. Stage A is steeply sloped to quickly accelerate the piston from its resting position, thereby taking advantage of the fact that linear proportional solenoid has a fast response and almos t no starting delay. Stage B is more moderately sloped to smoothly increase the driving force on piston 42, whereby a non -linear pressure increase is realized in plenum chamber 48. Stage C is a steeply sloped discharge stage which decreases the electromotive force applied to piston 42 back to zero, whereby the piston returns with solenoid plunger 57 back to a reference position under urging of a spring (not shown) located within the housing of linear proportional solenoid 52.

The waveform shown in Fig. 3 is achieved using a lookup table having only nine data points. These data points correspond to the slope, the start time, and the stop time of each of the three line segments corresponding to stages A, B, and C. The data is converted to analog voltage signal form by a digital -to- analog converter 62 either on -board or separate from microprocessor 56, and the voltage signal is applied to current source 54. Fig. 4 provides a conceptual schematic diagram of current source 54, which operates by controlling the charge and discharge current in a capacitor 66. Current source 54 comprises a pair of voltage-to-current converter circuits 64, one for charge and the other for discharge.

As will be appreciated from the foregoing description, the look -up table values stored in programmable memory 60 can be adjusted to provide customized control of the piston stroke. For example, the current can be held constant for a period of time at the end of the stroke at a current level that achieves equilibrium between the el ectromotive drive force and the return spring force, such that the plunger 57 and piston 42 are held in place. Then the current can be slowly decreased to allow piston 42 and plunger 57 to return in a controlled manner to the starting reference position. This manner of control helps minimize undesirable drawback of eye fluids into the discharge tube 20.

## Claims

1. A non-contact tonometer having a cylinder defining a compression chamber for compressing fluid within the compression chamber, a piston mova ble in a forward direction along a stroke axis relative to the cylinder, drive means operatively connected to the piston for forcing the piston in the forward direction, **characterized in that**, the drive means comprises a linear proportional solenoid.

2. A non-contact tonometer according to Claim 1, **characterized in that**, an energizing means for supplying current to the drive means is provided.

3. A non-contact tonometer according to Claim 2, **characterized in that**, the energizing means is controlled by a m icroprocessor.

4. A non-contact tonometer according to one of the Claims 2 or 3, **characterized in that**, the energizing means supplies the current according to a programmable look -up table.

5. A non-contact tonometer according to Claim 4, **characterized in that**, the programmable look-up table includes data as to the slope, start time, and stop time of one or more linear segments defining a current versus time relationship.

6. A non-contact tonometer according to one of the Claims 1 to 5, **characterized in that**, the non -contact tonometer has a fluid discharge tube in flow communication with the compression chamber for directing a fluid pulse along a test axis.

7. A fluid pump system, especially for a non -contact tonometer, having a cylinder defining a compression chamber for compressing fluid within the compression chamber, a piston movable in a forward direction along a stroke axis relative to the cylinder, drive means operatively connected to the piston for forcing the piston in the forward direction and e nergizing means for supplying current to the drive means, **characterized in that**, the drive means comprises a linear proportional solenoid.

8. A fluid pump system according to Claim 7, **characterized in that**, the energizing means is controlled by a micropro cessor.

9. A fluid pump system according to one of the Claims 7 or 8, **characterized in that**, the energizing means supplies the current according to a programmable look-up table.

10. A fluid pump system according to Claim 9, **characterized in that**, the programmable look-up table includes data as to the slope, start time, and stop time of one or more linear segments defining a current versus time relationship.
